# EUROPEAN PATENT APPLICATION

(11) **EP 3 756 644 A1**
(43) Date of publication of application: **30.12.2020**
(21) Application number: 20182385.3
(22) Date of filing: 25.06.2020
(51) Int. Cl.: A61K 8/34, A61Q 19/08

(54) **COMPOSITIONS AND METHODS FOR TREATING SKIN CONDITIONS USING INFRARED LIGHT AND RESORCINOLS**

(30) Priority: 25.06.2019 US 201916451762
(71) Applicant: JOHNSON & JOHNSON CONSUMER INC., Skillman, NJ 08558 (US)
(72) Inventor: FASSIH, Ali, Skillman, NJ New Jersey 08558 (US); LI, Wen-Hwa, Skillman, NJ New Jersey 08558 (US); PARSA, Ramine, Skillman, NJ New Jersey 08558 (US); SOUTHALL, Michael D., Skillman, NJ New Jersey 08558 (US)
(74) Representative: Kirsch, Susan Edith

(57) **Abstract**

The present invention provides compositions, methods and kits for treating skin, which combine administration of a substituted resorcinol and infrared light having a peak wavelength of about 750 nm to about 1000 nm.

## Description

### FIELD OF THE INVENTION

Aging is a universal process marked by a progressive decline in the function of multiple cells and tissues. Skin aging is a result of the combination of photoaging and chronological aging. Chronological aging represents the physiological changes in skin that occur over time. Photoaging results from the damaging effects of solar UV radiation, causing the skin to age prematurely. Aged skin is characterized by thin skin associated with wrinkles, laxity, uneven tone and dryness.

At the molecular level, skin aging is associated with the deterioration of cellular processes including cellular senescence. Cellular senescence is a cellular process where cells cease dividing and undergo distinctive phenotypic alterations, such as profound chromatin changes. Lamin B1 is a structural component of the cell nucleus, and loss of lamin B1 is associated with cell senescence. It has been reported that loss of lamin B1 is a robust marker to detect and quantify senescence in skin cells associated with skin aging.

Many products and methods have been proposed for combating signs of skin aging, including products containing substituted resorcinols such as hexylresorcinol. These are known to provide benefits to the skin when applied topically. For example, NEUTROGENA's Triple Age Repair Moisturizer Broad Spectrum SPF 25, commercially available from Johnson & Johnson Consumer Inc., contains hexylresorcinol and is marketed for anti-aging benefits.

US Patent No. 8,318,217 relates to compositions comprising a blend of an NFk-B inhibitor, which may be a substituted resorcinol such as 4-hexylresorcinol, and an anti-inflammatory compound having a low IC50. The patent discloses that substituted resorcinols may be used in the composition in a safe and effective amount, such as from about 0.01% to about 10%, preferably from about 0.1% to about 5%, more preferably from about 0.2% to about 2%, even more preferably about 1%, by weight of the composition. The use of resorcinols is also disclosed for example in US Patent Nos. 4,959,393 and 6,863,897 and US Published Patent Application No. 2008/0305059.

Light therapy is also known to be effective for treating skin conditions, and it is known that red light having a wavelength of about 633 nm has an anti-inflammatory effect. The mechanism of anti-inflammation action of red light is thought to occur by inhibition of inflammatory mediators and enzymes such as interleukin-1a and matrix metalloproteinases.

US 8,771,328 discloses improved phototherapy systems comprising a therapeutic lamp platform for radiant lamps such as LED's disposed in a convenient device that may be in the form of a facial mask. The system emits different wavelengths of radiant energy, for example at least two of blue, red, or infrared.

The illuMask® Anti-Aging Light Therapy Mask sold by La Lumiere, LLC emits red and infrared light to treat aging skin.

US 7,066,941 relates to the treatment of aging or damaged skin by irradiating it with an effective amount of visible light having a wavelength of about 400 nm to about 500 nm. The light source may be sunlight or artificial light for example, and in one embodiment, light-emitting diodes are applied to discrete skin areas. Compositions containing compounds that enhance light penetration of the stratum corneum such as alpha-hydroxy acids and/or filter light may be applied to the skin prior to or during phototreatment.

WO 2016/146778 relates to cosmetic methods of providing skin care comprising illuminating the skin of a subject with one or more light beams that provide light to the skin having a discontinuous spectrum with peaks in wavelengths corresponding to green light, red light, and infrared light along with a topical composition that may contain a wide variety of ingredients, including glucosamine. Such methods are said to be beneficial for skin activation processes involving proliferation and migration of skin cells and production of extracellular matrix fibers such as collagen.

Applicants have now discovered that the production of lamin B1 by skin may be surprisingly upregulated by application of a topical composition comprising about 0.01 to about 5 weight percent of at least one substituted resorcinol and exposure of said skin to infrared light using a light delivery device, thus providing significant and unexpected benefits for skin, including improving, reducing, inhibiting, or delaying the appearance of at least one sign of aging in skin. The combination may also enhance skin barrier protection and skin moisturization. Accordingly, new methods of treating signs of skin aging and moisturizing skin, for example, are now available.

### SUMMARY OF THE INVENTION

The present invention provides a method of increasing the production of lamin B1 by skin, comprising topically applying to skin in need of increased lamin B1 a topical composition comprising about 0.01 to about 5 weight percent of a substituted resorcinol and exposing said skin to infrared light having a peak wavelength of about 750 nm to about 1000 nm using a light delivery device.

The present invention also provides a method of treating skin, comprising topically applying to skin in need of treatment for signs of skin aging a topical composition comprising about 0.01 to about 5 weight percent of a substituted resorcinol and exposing said skin to infrared light having a peak wavelength of about 750 nm to about 1000 nm using a light delivery device.

The present invention further provides a method of treating skin, comprising topically applying to skin in need of moisturization a topical composition comprising about 0.01 to about 5 weight percent of a substituted resorcinol and exposing said skin to infrared light having a peak wavelength of about 750 nm to about 1000 nm using a light delivery device.

The invention also provides a kit comprising: (a) a topical composition comprising about 0.01 to about 5 weight percent of a substituted resorcinol, and (b) a light delivery device that delivers infrared light having a peak wavelength of about 750 nm to about 1000 nm.

### DETAILED DESCRIPTION OF THE INVENTION

Unless defined otherwise, all technical and scientific terms used herein have the same meaning as commonly understood by one of ordinary skill in the art to which the invention belongs. All publications, patent applications, patents, and other references mentioned herein are incorporated by reference.

As used herein, "topically applying" means directly laying on or spreading on outer skin or the scalp, e.g., by use of the hands or an applicator such as a wipe, roller, or spray.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically effective amount" means an amount of a physiologically active compound or composition sufficient for treating one or more conditions, but low enough to avoid serious side effects. The cosmetically effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

As used herein, "cosmetically acceptable" means that the ingredients the term describes are suitable for use in contact with tissues (e.g., the skin) without undue toxicity, incompatibility, instability, irritation, allergic response, or the like.

As used herein, a "cosmetically acceptable active agent" is a compound (synthetic or natural) that has a cosmetic or therapeutic effect on the skin.

As used herein, "treatment or treating" refers to mitigating, reducing, preventing, improving, or eliminating the presence or signs of a condition or disorder.

The present invention is suitable for treating signs of skin aging. As used herein, "signs of skin aging" includes the presence of lines and wrinkles, loss of elasticity, uneven skin, and blotchiness. In a particularly preferred embodiment, the sign of aging is the presence of lines and wrinkles and/or loss of elasticity.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

Compositions of the invention are also useful for treating skin in need of moisturization. As used herein, "skin in need of moisturization" means a skin that is, but not limited to, lacking in moisture, lacking in sebum, cracked, dry, itchy, scaly, xerodermic, dehydrated, lacks suppleness, lacks radiance, dull, or lacks lipids.

Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

### Topical Composition Comprising a Substituted Resorcinol

The invention utilizes a topical composition comprising at least one substituted resorcinol. The composition may comprise one or more than one substituted resorcinol.

In one embodiment, the composition comprises about 0.01 to about 5 weight percent of the substituted resorcinol. In another embodiment, the composition comprises about 0.1 to about 3 weight percent of the substituted resorcinol.

Resorcinol is a dihydroxy phenol compound (i.e., 1,3-dihydroxybenzene), having the following structure:

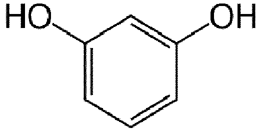

The resorcinols used herein are "substituted resorcinols." As used herein, "substituted resorcinol" means resorcinol comprising at least one substituent in the 2, 4, 5, or 6 position. Thus, the resorcinol may have as few as one and as many as four substituents. Positions 1 and 3 of the resorcinol preferably comprise an -OH group as shown above; however, one or both -OH groups may be replaced by an -OR group in which R is a C₁-C₁₂ alkyl or acyl group.

In certain preferred embodiments, at least one substituent on the resorcinol comprises 2 to 18 carbon atoms, preferably 2 to 12 carbon atoms, more preferably 5 to 10 carbon atoms, even more preferably 5 to 9 carbon atoms, most preferably 5 to 8 carbon atoms. In certain other embodiments, at least one substituent comprises a (linear or branched) alkyl group, such as one having the number of carbon atoms described above. Preferably, at least one substituent comprises an alkyl group that is unsaturated and linear.

In certain embodiments, the 4 position of the resorcinol is substituted, and, in certain embodiments, only the 4 position is substituted. In another embodiment, the 4 position is substituted with an akyl group. In certain preferred embodiments, the resorcinol comprises a single substituent at the 4 position that comprises an alkyl group. In certain other preferred embodiments, the resorcinol comprises a single substituent at the 4 position that consists of an alkyl group directly bonded to the benzene ring.

Particularly suitable substituted resorcinols include 4-hexyl resorcinol and 4-octylresorcinol, particularly 4-hexyl resorcinol. The structures of 4-hexylresorcinol and 4-octylresorcinol are shown below:

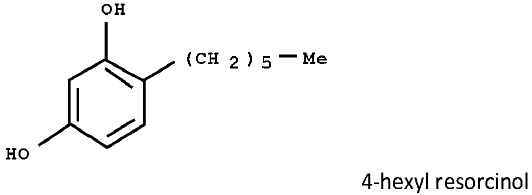

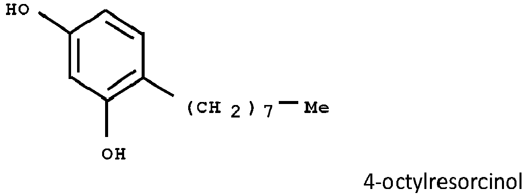

4-Hexyl resorcinol is commercially available as "SYNOVEA HR" from Sytheon of Lincoln Park, NJ. It is also commercially available from Alfa Aesar, Tewksbury, MA. 4-Octylresorcinol is commercially available from City Chemical LLC of West Haven, Connecticut.

The composition may optionally comprise a wide variety of additional oil-soluble materials and/or water-soluble materials conventionally used in compositions for use on skin, at their art-established levels. For example, surfactants, pearlescent or opacifying agents, thickeners, emollients, conditioners, humectants, chelating agents, exfoliants, and additives that enhance the appearance, feel, or fragrance of the cleansing composition, such as colorants, fragrances, preservatives, pH adjusting agents, and the like, can be included.

The pH may be measured by a Corning pH meter 430. The pH may be measured at room temperature (25°C).

The composition may comprise one or more other cosmetically acceptable active agents include for example anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, and agents for skin conditioning.

The amount of other cosmetically active agent in may range from about 0.001% to about 20% by weight of the composition, e.g., about 0.005% to about 10% by weight of the composition, such as about 0.01% to about 5% by weight of the composition.

The cosmetically acceptable active agent may be selected for instance from salicylic acid, succinic acid, benzoyl peroxide, D-panthenol carotenoids, ceramides, polyunsaturated fatty acids, essential fatty acids, enzymes such as laccase, enzyme inhibitors, minerals, hormones such as estrogens, steroids such as hydrocortisone, 2-dimethylaminoethanol, copper salts such as copper chloride, peptides like argireline, syn-ake and those containing copper, coenzyme Q10, amino acids such as proline, vitamins, lactobionic acid, acetyl-coenzyme A, niacin, riboflavin, thiamin, ribose, electron transporters such as NADH and FADH2, natural extracts such as from aloe vera, feverfew, oatmeal, dill, blackberry, princess tree, *Picia anomala,* and chicory, curcuminoids, sugar amines such as N-acetyl glucosamines, and derivatives and mixtures thereof.

Examples of vitamins include, but are not limited to, vitamin A, vitamin B's such as vitamin B3, vitamin B5, and vitamin B12, vitamin C, vitamin K, and different forms of vitamin E like alpha, beta, gamma or delta tocopherols or their mixtures, and derivatives thereof.

Examples of antioxidants include, but are not limited to, water-soluble antioxidants such as sulfhydryl compounds and their derivatives (e.g., sodium metabisulfite and N-acetylcysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

The composition may further include a cosmetically acceptable topical carrier. The carrier may be from about 50% to about 99.99%, by weight, of the composition (e.g., from about 80% to about 99%, by weight, of the composition). In one embodiment of the invention, the cosmetically acceptable topical carrier includes water.

The composition may be made into a wide variety of product types that include but are not limited to lotions, creams, gels, sticks, sprays, ointments, pastes, foams, powders, mousses, creams, wipes, patches, hydrogels, film-forming products, facial masks and skin masks, dissolving or non-dissolving films, and make-up such as foundations. These product types may contain a variety of cosmetically acceptable topical carriers including, but not limited to solutions, suspensions, emulsions such as microemulsions and nanoemulsions, gels, solids, films and liposomes. The following are non-limiting examples of such carriers. Other carriers can be formulated by those of ordinary skill in the art.

The composition can be formulated as a solution. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

The composition may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

The composition alternatively be anhydrous or be an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically, such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The composition can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The composition can also be formulated into a solid (e.g., wax-based stick, bar, or powder).

The composition may be contained in a substrate, such as a film, woven or non-woven material, wipe, patch, mask, article of clothing and the like.

In one embodiment, the composition is contained in a film. As used herein, the term "film" means a composition that forms a thin layer or membrane on mammalian, and more particularly human skin. Such film may comprise a single layer or multiple layers.

In one embodiment, the film is a dissolvable film. A variety of dissolvable films are known in the art, and any one of these may be used according to the invention.

In one particular embodiment, the film may comprise an integral film product as described in US 2015/0182991, the disclosure of which is incorporated herein by reference. The integral film product is arranged and configured to be removable from the manufacturing substrate it is made on, for use independent of the manufacturing substrate. In particular, the product may be made by placing a mask over a manufacturing substrate having a releasable surface, delivering a film-forming composition through the mask to form a raw shape on the manufacturing substrate; removing the mask; and solidifying the raw shape into the integral film product disposed on the manufacturing substrate. The mask has at least one aperture having a shape corresponding to the desired integral film product. The integral film product is arranged and configured to be removable from the releasable surface of the manufacturing substrate for use independent thereof.

In another embodiment, the film is a multilayered shaped film product as described in US 2015/0182990, the disclosure of which is incorporated herein by reference. For example, a two layer shaped film product comprising a first surface comprising the topical composition to be delivered to skin, and a second surface exposed to the exterior, may be used. Such an article of manufacture may be made using a process that comprises delivering liquid film-forming compositions through a mask; removing the mask to leave a multilayered raw shape; and curing the multilayered raw shape to form the multilayered shaped film product. The mask has a delivery surface, an opposite surface and at least one aperture having a design corresponding to the desired shaped film product. The film-forming compositions are delivered through a multistream nozzle. The movement of the mask and the delivery of the first and second liquid film-forming compositions to the mask aperture are controlled to provide a volumetric flow rate of the first and second liquid film-forming compositions to the mask aperture corresponding to the volume of a void. The nozzle is in contact with the delivery surface of the mask.

In another particular embodiment, the film may be multilayered film product as described in US 2015/0182992, the disclosure of which is incorporated herein by reference. For example, a two layer shaped film product in which a first layer has a larger surface area than a second layer disposed on the first layer may be used. This forms an "island" of the second layer on top of the first layer. One of the two layers is for contacting the skin and comprises the composition of the invention. The other layer is exposed to the exterior. Such an article of manufacture may be made by a process that comprises delivering a first film-forming composition through a first mask to form a first raw shape; removing the first mask; placing a second mask over the first raw shape; delivering a second film-forming composition through the second mask to form a second raw shape on the first raw shape; removing the second mask; and solidifying the first and second raw shapes to provide a shaped film product.

In a further embodiment, a shaped film product as described in US 2015/0182993, the disclosure of which is incorporated herein by reference, may contain the composition. Such shaped film product may be made by placing a mask over a manufacturing substrate; delivering a film-forming composition through a nozzle to form a raw shape on the manufacturing substrate; removing the mask; and solidifying the film-forming composition to provide the shaped film product disposed on the manufacturing substrate. The mask has a delivery surface and an opposite manufacturing substrate-facing surface and at least one aperture having a design corresponding to the desired shaped film product. The nozzle is disposed in sealing engagement with the delivery surface of the mask to the at least one aperture of the mask during delivery of the film-forming composition.

In yet another embodiment, a multilayer topically applied film as described in US 2016/0367490, the disclosure of which is incorporated herein by reference, may be used. This film is readily removable upon application of water thereto. As used herein, "readily removable" means the film may dissolve or disintegrate upon application of water to the film, such that it may be removed from the skin without scrubbing or the like.

Such a film comprises a first top layer having a first top surface for facing outwardly from the skin and a first bottom surface opposite the first top surface for facing towards the skin. The article also comprises a bottom skin-contacting layer comprising a second top surface facing and adhered to the first bottom surface of the first top layer and a second bottom surface that is outwardly-facing for contacting and adherence of the article to the skin when the article is applied thereto. The bottom skin-contacting layer comprises the topical composition. In addition, each of the first top layer and second bottom layer comprises a water-soluble film former and the article is readily removable from the skin upon application of water thereto.

This film containing the topical composition may be formed by one of the above-described processes of forming multilayer shaped film products. It may also be made by casting and drying an adhesive layer, and then casting the top layer on top of the bottom layer. The two layers may adhere to one another by any of the known methods of adhesion (mechanical, chemical, dispersive, electrostatic, diffusive, etc.). In one embodiment, the two layers preferably are both water soluble, so that the water in the non-adhesive outwardly-facing layer will slightly dissolve the already dried adhesive skin-contacting layer, thereby creating a certain amount of diffusive adhesion at the interface of the two layers. In a second embodiment, both layers are cast wet on wet, and intermixing of the materials occurs at their interface, thereby creating a bond by diffusive adhesion. Preferably, the materials have a common solvent and / or are miscible with each other so that they intermix and bond together. It will be appreciated that the materials of the adhesive and non-adhesive layers (the skin--contacting and outwardly-facing layers, respectively) may have a common solvent other than water, such as alcohol, so that the materials bond to each other.

For example, the skin-contacting layer preferably comprises a hydrophilic film-forming polymer, a solubilizing agent to solubilize other ingredients in the film, a disintegration promoter, a thickening agent/ structuring agent/ texture modifier, a hydroscopic agent/wetting agent to retain skin moisture, a partition coefficient modifier/ absorption-or permeation-promoting substances to drive the hydroscopic agent into skin, a plasticizer/primary adhesive agent for flexibility and softness, a solvent used for hydrocolloids and retain latent moisture and keep final article flexible and other auxiliaries or additives. The skin-contacting layer is applied preferably directly to the skin surface and possesses properties suitable for use as the skin-contacting surface of the article. Such properties include rapid dissolution, sustained adhesion strength, semi-occlusiveness, and flexibility. The skin-contacting layer comprises the glucosamine hydrochloride and other ingredients of the topical composition.

The outwardly-facing layer possesses proprieties suitable for use as a physical barrier, allowing it to remain clean of dust and dirt and debris while the article remains in place on the application site. Such proprieties include rapid dissolution, semi-occlusiveness, flexibility, and non-stickiness. The outwardly-facing layer comprises a hydrophilic film forming polymer, a disintegration promoter, an oil-in-water emulsifier, a wax to limit water migration from the skin-contacting layer to the topical layer, a plasticizer for flexibility and softness, a primary adhesive agent, a solvent used for hydrocolloids and to retain latent moisture and to keep the final article flexible, and other auxiliaries or additives.

In a particular embodiment of the invention, the topical composition is contained in such a multilayer, water-removable film. The film may have a thickness, for example, of up to about 2 mm. The film is placed on the skin by adhering the second bottom surface to the skin. The film is then exposed to IR light using a light delivery device according to the invention. The film is maintained in place for a period of time, for example, at least 15 minutes, or at least 30 minutes, or at least 3 hours, or at least 6 hours, whereby the substituted resorcinol is capable of transferring to the skin application site. The film is then removed from the application site by application of water, whereupon the film dissolves.

In a further embodiment of the above, the bottom skin-contacting layer further comprises an effective amount of an emulsifier to enhance transport of the substituted resorcinol to the skin. In one embodiment, the emulsifier is a glycerine derivative. For instance, the emulsifier may be selected from the group consisting of glycerides and glycerol fatty acid esters.

### Light Delivery Device

The light delivery device may comprise any source of infrared light.

As used herein, "infrared" or "IR" light means light having a peak wavelength of about 750 to about 1000 nm, preferably about 800 to about 900 nm. In one embodiment, the infrared light has a peak wavelength of about 830 nm.

The peak wavelength may be measured by an Optronic OL770 VIS/NIR spectroradiometer, 380 nm to 1100 nm. Measurements may be carried out at room temperature (25°C).

The light delivery device may take any form or configuration, provided it emits infrared light. The light may be delivered continuously, pulsed, focused, diffuse, multi-wavelength, coherent, or non-coherent within the desired range, or at the desired wavelength(s).

In certain preferred embodiments, only infrared light is delivered to the skin. That is, light of other wavelengths is not delivered to the skin, either by filtering of such other wavelengths or the absence of such wavelengths in the light delivered by the light delivery device.

The light is preferably delivered at low intensity. In one embodiment, the intensity of the light is below about 1 mW/cm². In another embodiment, the intensity of the light is about 0.5 mW/cm² and it is delivered for about 10 minutes.

The light intensity may be measured by a Newport light sensor (818-ST2/DB) and a Newport power meter 1936-R. The light intensity may be measured at room temperature (25°C).

The light source may be for example one or more LEDs. The LEDs may be for example individual LED bulbs or multi-LED strips.

The device may be in the form of a shaped mask, shroud, or hood for use on the face. Alternatively, the device may be shaped for use on the body, in particular the torso, such as a shirt, vest, or the like. The device may be in the form or a patch having a circular, oval, rectangular, or other shape. Such a patch may also have an irregular shape, or a shape designed to fit a particular part of the face or body.

In one embodiment, the device comprises a lamp platform and remote battery pack as described in US 8,771,328, the disclosure of which is incorporated by reference herein. The lamp platform for radiant lamps such as LEDs are disposed in an assembly comprising a first wall to which the lamps are affixed thereto and a second wall, closer to the skin, spaced from the first wall wherein the lamps are recessed relative thereto. The second wall comprises a reflective surface facing towards the skin and a plurality of light apertures substantially aligned with the LEDs on the first wall for communicating lamp radiation from the lamps to a user. The lamps and associated circuitry are disposed between the first and second wall so that the reflective surface is relatively smooth and seamless towards the skin. The number of lamps are minimized, as is the circuitry therefor, and other assembly materials are purposefully selected for a relatively light weight assembly resulting in enhanced user comfort during therapy sessions. The walls have a malleable rigidity for flexible adjustability relative to the user. More particularly, the walls have a concave configuration relative to the face of the user which is adjustable relative to a rest position to be expandable relative to a size of the head of the user for a close fitting and secure engagement to the user during use. The device is mounted to the user with a frame comprising an eyeglass frame or goggles including lenses for shielding the user's eyes from lamp radiation. The adjustability of the embodiments is further enhanced by the walls being pivotable relative to the support frame and where the frames may include telescopic temple arms for selective adjustability relative to the head size of the user. The device is thus supported on the patient as a wearable hands-free mask or the like. A power source communicates energy to the lamps and comprises a remote battery pack and may also include a control processor for counting the number of uses by the device for the user and for indicating a need for device replacement after a predetermined number of uses.

The platform can be secured to the head by multiple means: eyeglass frames, straps, drawstring, harness, VELCRO, turn dial or snap and buttons. As the mask is secured it can be adjusted upward, for chin to forehead coverage. It can also be adjusted outward, for side-to-side coverage. In addition, once the platform has been bent/slid to cover the face area, the distance of the platform from the skin can be adjusted for achieving a desired light intensity relative to a user's skin surface. Thus, the light therapy can be maximized in up to three physical dimensions.

The subject adjustability may be implemented through "smart" processing and sensor systems for enhanced flexibility/adjustability in the form of adjustable energy output, adjustable wavelengths, priority zones, timers, and the like. The sensors of the sensor systems will enable the subject embodiments to have the ability to evaluate the skin of the face and body of a patient with sensors for color, acne, lesion density, and the like, and plan a smart treatment, utilizing more or less energy on the priority zones. The subject embodiments can be smart from the standpoint of skin type, age, overall severity of problems and have the ability to customize the treatment accordingly.

In another embodiment, the device comprises a therapeutic lamp platform for radiant lamps such as LED's disposed in a holdable spot applicator assembly, as described in US 2016/0045758, the disclosure of which is incorporated by reference herein. The holdable spot applicator assembly includes a reflective surface facing towards a patient and a plurality of LED's for communicating lamp radiation from the lamps to a user. The lamps and the associated circuitry are housed within a holdable elongated structure.

In one embodiment, ultrasonic energy is also delivered to the skin, concurrently or in series with the light. The ultrasonic energy may be delivered by the light device or by a separate device.

In one embodiment, the light delivery device delivers both light and ultrasonic energy.

### Production of Lamin B1

According to the invention, a combination of topical administration of substituted resorcinol and exposure to infrared light to aging skin provides an unexpected increase in the amount of lamin B1 produced by such skin. It has been found that such combinations provide a synergistic boost in the amount of lamin B1 produced by aging skin relative to the amount produced by either topical administration of a substituted resorcinol or exposure to infrared light alone.

In one embodiment, the combination provides at least a 1.5 fold increase in lamin B1 production.

Lamin B1 production may be measured by gene expression assays, as known in the art. For example, lamin B1 production may be measured by the gene expression assay set forth in Example 1 using skin equivalents and TNF-α to simulate photoaged skin.

The topical composition and the light may be administered to the skin simultaneously or sequentially. When administered sequentially, the composition and light may be administered in either order. When administered with ultrasonic energy as well, the composition, light, and ultrasonic energy may be administered simultaneously or in any order.

In one embodiment of the invention, skin in need of treatment for signs of skin aging is treated by topically applying to the skin a composition comprising about 0.01 to about 5 weight percent of substituted resorcinol and exposing the skin to infrared light having a peak wavelength of about 750 nm to about 1000 nm.

In another embodiment of the invention, skin in need of treatment for moisturization is treated by topically applying to the skin a composition comprising about 0.01 to about 5 weight percent of substituted resorcinol and exposing the skin to infrared light having a peak wavelength of about 750 nm to about 1000 nm using a light delivery device.

The following non-limiting examples further illustrate the invention.

### Example 1

The effects of IR light, hexyl resorcinol (HR), and combinations thereof on lamin B1 gene expression in the presence of TNF-α was tested as follows. TNF-α is known to play a primary role in the inflammation process upon ultraviolet (UV) irradiation and is stimulated by UVB.

Three human full-thickness skin equivalents (MatTek, Ashland, MA) were used for each treatment. The skin equivalents were treated with one of the following: (i) 100 ng/ml TNF-α in a medium (1X phenol-red free Hank's buffered saline solution (HBSS), (ii) a placebo formulation, (iii) a hexyl resorcinol formulation containing 0.5 weight percent hexyl resorcinol (HR) in the placebo formulation, (iv) infrared (IR) light (wavelength 830 nm) at 0.5 mW/cm² for 10 min (i.e. fluence at 0.3 J/cm²), or (iv) combinations of these. TNF-α was used to simulate photoaged skin.

The placebo formulation contained the ingredients in Table 1. The hexyl resorcinol formulation contained the same ingredients plus 0.5 wt% hexyl resorcinol.

**TABLE 1**

| |
|---|
| water |
| glycerine |
| dimethicone |
| isononyl isononanoate |
| ethylhexyl palmitate |
| propylene glycol |
| steareth-2 |
| butyrospermum sparkii (shea) butter |
| methyl methacrylate crosspolymer |
| steareth-21 |
| ammonium acryloyldimethyltaurate/VP copolymer |
| ascorbyl glucoside |
| sclerotium gum |
| xylitol |
| xylithylglucoside |
| anhydroxylitol |
| disodium EDTA |
| stearyl alcohol |
| arachidyl alcohol |
| cetyl alcohol |
| lignoceryl alcohol |
| sodium hycroxide |
| phenoxyethanol |
| methylparaben |
| propylparaben |
| ethylparaben |
| titanium dioxide |
| Mica* |
| fragrance |

| |
|---|
| *the mica (CAS number 12001-26-2) has the chemical formula H₄Al6K₂O₂₄Si₆ |

For the combination treatments, the formulations were first topically applied to the skin equivalents and left to stand for two hours. Next, the skin equivalents were irradiated with the IR light. Then the skin equivalents were incubated with or without 100 ng/ml TNF-α in the phenol-red-free medium for 24 hours.

Afterwards, the skin equivalents were collected in RNAlater solution (ThermoFisher Scientific, Bridgewater, NJ, USA) for RNA purification. The total RNAs were purified using a Qiagen RNeasy kit (Valencia, CA, USA) following the manufacturer's instructions. RNA concentration was assessed using a Nanodrop 2000 spectrophotometer (ThermoFisher Scientific, Bridgewater, NJ, USA). Complementary DNA (cDNA) was performed using a High Capacity cDNA kit (ThermoFisher Scientific, Bridgewater, NJ, USA). TaqMan® gene expression assays for lamin b1 (LMNB1) and TATA-Box Binding Protein (TBP) and TaqMan® gene expression master mix from ThermoFisher Scientific (Bridgewater, NJ, USA) were used. Realtime polymerase chain reactions (qPCRs) of each treatment were performed using 5 ul of the 15 ul mixture of cDNAs pooled from three equivalent tissue replicates (i.e. mixing 5 ul of cDNA from each equivalent replicate). qPCRs were performed using a QuantStudio™ 7 Flex System (ThermoFisher Scientific, Bridgewater, NJ, USA). The RQ (relative quantitation) was calculated using the formula 2^-ΔΔCt.

The results are shown in Table 2.

**TABLE 2**

| **Treatment** | **LMNB1 Induction (RQ**) | **Difference of Induced LMNB1 vs Untreated Control (RQ fold changes)** |
|---|---|---|
| Untreated | 1 | - |
| Placebo formulation | 0.83 | -0.17 |
| HR formulation (6 ul, Topical) | 1.16 | 0.16 |
| TNF-α (100ng/ml, medium) | 1.55 | 0.55 |
| IR Light (0.3 J/cm², topical) | 0.67 | -0.33 |
| HR formulation + TNF-α | 1.41 | 0.41 |
| HR formulation + IR | 1.12 | 0.12 |
| IR Light + placebo formulation + TN F-α | 1.48 | 0.48 |
| IR Light + HR formulation + TNF-α | 2.66 | 1.66 |

The results show that administration of a combination of a substituted resorcinol and IR light in the presence of TNF-α mimicking photoaged skin provided unexpected, synergistic and significant lamin B1 gene induction compared with either treatment alone. The treatment comprising a combination of a substituted resorcinol and IR light in the presence of TNF-α was the only one of all the treatments tested providing above a two-fold induction of lamin B1 gene expression.

### Example 2

The effects of red light (wavelength 640 nm, 0.3 J/cm²) and HR on lamin B1 gene expression in the presence of TNF-α was tested using the same method as Example 1.

The results are shown in Table 3.

**Table 3**

| **Treatment** | **Induced LMNB1 (RQ)** | **Induced LMNB1 vs Untreated Control (RQ fold changes)** |
|---|---|---|
| Untreated | 1 | 0 |
| Placebo cream | 0.83 | -0.17 |
| 0.5% HR Cream (6 ul, Topical) | 1.16 | 0.16 |
| TNF-α (100ng/ml, medium) | 1.55 | 0.55 |
| Red Light (0.3 J/cm², topical) | 1.23 | 0.23 |
| 0.5% HR Cream+ Red (0.3 J/cm², topical) | 1.14 | 0.14 |
| 0.5% HR Cream + TNF-α (100ng/ml, medium) | 1.41 | 0.41 |
| Red Light + placebo cream + TNF-α | 0.87 | -0.13 |
| Red Light + 0.5% HR Cream + TNF-α | 1.59 | 0.59 |

Surprisingly, no substantial change in lamin B1 gene expression was provided by administration of a combination of hexyl resorcinol and red light. Thus, synergistic upregulation of lamin B1 is unique to the combination of IR light with a substituted resorcinol.

A non-exhaustive list of aspects of the invention is provided:
1. A method of increasing the production of lamin B1 by skin, comprising topically applying to skin in need of treatment for signs of skin aging, a topical composition comprising about 0.01 to about 5 weight percent of a substituted resorcinol and exposing said skin to infrared light having a peak wavelength of about 750 nm to about 1000 nm using a light delivery device.
2. The method of aspect 1, wherein the topical composition comprises about 0.1 to about 3 weight percent of substituted resorcinol.
3. The method of aspect 1, wherein the topical composition has a pH of about 3.0 to about 5.5.
4. The method of aspect 1, wherein the intensity of the light is below about 20 mW/cm².
5. The method of aspect 1 further comprising exposing said skin to ultrasonic energy.
6. The method of aspect 5, wherein the light delivery device delivers light and ultrasonic energy.
7. A method of treating skin, comprising topically applying to skin in need of treatment for signs of skin aging a topical composition comprising about 0.01 to about 5 weight percent of a substituted resorcinol and exposing said skin to infrared light having a peak wavelength of about 750 nm to about 1000 nm using a light delivery device.
8. The method of aspect 7, wherein the topical composition comprises about 0.1 to about 3 weight percent of substituted resorcinol.
9. The method of aspect 7, wherein the signs of skin aging are fine lines and wrinkles.
10. The method of aspect 7, wherein the topical composition has a pH of about 3.0 to about 5.5.
11. The method of aspect 7, wherein the intensity of the light is below about 20 mW/cm².
12. The method of aspect 7 further comprising exposing said skin to ultrasonic energy.
13. The method of aspect 12, wherein the light delivery device delivers light and ultrasonic energy.
14. A method of treating skin, comprising topically applying to skin in need of moisturization a topical composition comprising about 0.01 to about 5 weight percent of a substituted resorcinol and exposing said skin to infrared light having a peak wavelength of about 750 nm to about 1000 nm using a light delivery device.
15. The method of aspect 14, wherein the topical composition comprises about 0.1 to about 3 weight percent of substituted resorcinol.
16. The method of aspect 14, wherein the signs of skin aging are fine lines and wrinkles.
17. The method of aspect 14, wherein the topical composition has a pH of about 3.0 to about 5.5.
18. The method of aspect 14, wherein the intensity of the light is below about 20 mW/cm².
19. The method of aspect 14 further comprising exposing said skin to ultrasonic energy.
20. The method of aspect 19, wherein the light delivery device delivers light and ultrasonic energy.
21. A kit comprising: (a) a topical composition comprising about 0.01 to about 5 weight percent of a substituted resorcinol, and (b) a light delivery device that delivers infrared light having a peak wavelength of about 750 nm to about 1000 nm.
22. The kit of aspect 21, wherein the topical composition comprises about 0.1 to about 3 weight percent of substituted resorcinol.

## Claims

1. A method of increasing the production of lamin B1 by skin, comprising topically applying to skin in need of treatment for signs of skin aging, a topical composition comprising about 0.01 to about 5 weight percent of a substituted resorcinol and exposing said skin to infrared light having a peak wavelength of about 750 nm to about 1000 nm using a light delivery device.

2. The method of claim 1, wherein the topical composition comprises about 0.1 to about 3 weight percent of substituted resorcinol.

3. The method of any of claims 1 or 2, wherein the topical composition has a pH of about 3.0 to about 5.5.

4. The method of any of claims 1 to 3, wherein the intensity of the light is below about 20 mW/cm².

5. The method of any of claims 1 to 4 further comprising exposing said skin to ultrasonic energy.

6. The method of claim 5, wherein the light delivery device delivers light and ultrasonic energy.

7. A method of treating skin, comprising topically applying to skin in need of:
(a) treatment for signs of skin aging, or
(b) moisturization,
a topical composition comprising about 0.01 to about 5 weight percent of a substituted resorcinol and exposing said skin to infrared light having a peak wavelength of about 750 nm to about 1000 nm using a light delivery device.

8. The method of claim 7, wherein the topical composition comprises about 0.1 to about 3 weight percent of substituted resorcinol.

9. The method of any one of claims 7 or 8, wherein the signs of skin aging are fine lines and wrinkles.

10. The method of any one of claims 7 to 9, wherein the topical composition has a pH of about 3.0 to about 5.5.

11. The method of any one of claims 7 to 10, wherein the intensity of the light is below about 20 mW/cm².

12. The method of any one of claims 7 to 11 further comprising exposing said skin to ultrasonic energy.

13. The method of claim 12, wherein the light delivery device delivers light and ultrasonic energy.

14. A kit comprising: (a) a topical composition comprising about 0.01 to about 5 weight percent of a substituted resorcinol, and (b) a light delivery device that delivers infrared light having a peak wavelength of about 750 nm to about 1000 nm.

15. The kit of claim 14, wherein the topical composition comprises about 0.1 to about 3 weight percent of substituted resorcinol.
